# EUROPEAN PATENT SPECIFICATION

(11) **EP 1 703 858 B1**
(45) Date of publication and mention of the grant of the patent: **21.10.2009**
(21) Application number: 04811411.0
(22) Date of filing: 19.11.2004
(51) Int. Cl.: A61F 2/06

(54) **COMPOSITE STENT WITH INNER AND OUTER STENT ELEMENTS AND METHOD OF USING THE SAME**
KOMPOSITSTENT MIT INNEREN UND ÄUSSEREN STENTELEMENTEN UND ANWENDUNGSVERFAHREN
STENT COMPOSITE COMPRENANT DES ÉLÉMENTS INTTÉRIEUR ET EXTÉRIEUR,AINSI QUE MÉTHODE D'UTILISATION

(30) Priority: 25.11.2003 US 720176
(43) Date of publication of application: 27.09.2006
(73) Proprietor: Scimed Life Systems, Inc., Maple Grove, MN 55311 (US)
(72) Inventor: Shank, Peter, J., Boylston, MA 01505 (US); Headley, Anthony, F., Jr., Marlborough, MA 01752 (US)
(74) Representative: Altenburg, Udo
(86) International application number: PCT/US2004/038695
(87) International publication number: WO 2005/053576

(56) References cited:
- US-A- 5 797 949
- US-A- 5 961 547
- US-A1- 2002 165 601
- US-B1- 6 214 040
- US-B1- 6 254 632
- US-B1- 6 451 050
- US-B1- 6 626 939

## Description

### 1. Field of the Invention

The present invention relates to body implantable treatment devices, and more particularly to stents and other prostheses intended for fixation in body lumens.

### 2. Description of Related Art

Medical prostheses frequently referred to as stents are well known and commercially available. These devices are used within body vessels of humans for a variety of medical applications. Examples include intravascular stents for treating narrowing or contraction of body lumens (stenoses), stents for maintaining openings in the urinary biliary, tracheobronchial, esophageal, and renal tracts, and vena cava filters. Stents may also be used by physicians for the treatment of benign and malignant tumors.

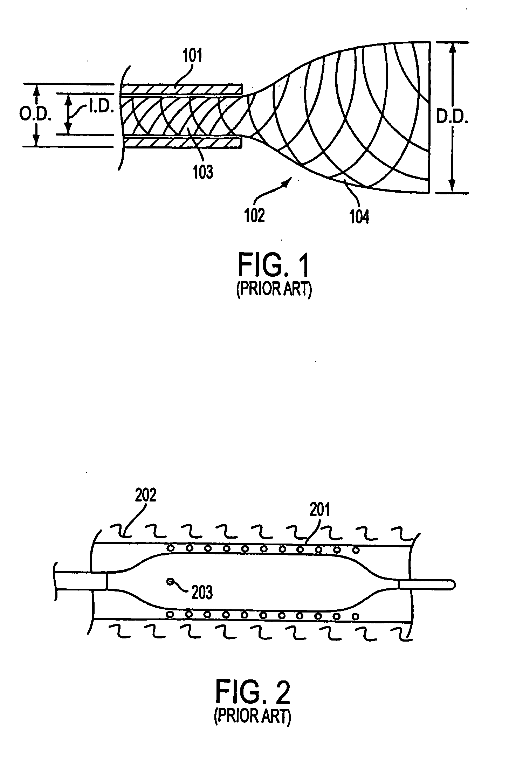

Typically, a stent is delivered into position at a treatment site in a compressed state using a delivery device. After the stent is positioned at the treatment site, the delivery device is actuated to release the stent. Following release of the stent, self-expanding stents are allowed to self-expand within the body vessel or lumen. Figure 1 shows such a configuration including a delivery device in the form of catheter 101 containing a portion 103 of self-expanding stent 102 within a lumen of the catheter having an outside diameter O.D. and an inside diameter I.D. Having exited an open distal end of the lumen, deployed portion 104 of stent 102 is shown expanding to a deployed diameter D.D. Alternatively, a balloon may be used to expand stents. This expansion of the stent in the body vessel helps to retain the stent in place and prevents or reduces movement or migration of the stent. Figure 2 shows stent 201 being expanded within a body lumen 202. A Percutaneous Transluminal Angioplasty (PTA) or Transluminal Coronary Angioplasty (PTCA) balloon 203 is inflated to expand stent 201 and urge it into position against body lumen 202.

Stents are typically composed of stent filaments, and may be categorized as permanent, removable or bioabsorbable. Permanent stents are retained in place and incorporated into the vessel wall. Removable stents are removed from the body vessel when the stent is no longer needed. A bioabsorbable stent may be composed of, or include bioresorbable material that is broken down by the body and absorbed or passed from the body after some period of time when it is no longer needed.

Commonly used materials for stent filaments include Elgiloy^{®} and Phynox^{®} metal spring alloys. Other metallic materials that may be used for stents filaments are 316 stainless steel, MP35N alloy and superelastic Nitinol nickel-titanium. Another stent, available from Schneider (USA) Inc. of Minneapolis, Minnesota, has a radiopaque clad composite structure such as shown in U.S. Patent No. 5,630,840 to Mayer. Stents can also be made of a titanium alloy as described in U.S. Patent No. 5,888,201.

Bioabsorbable implantable endoprostheses such as stents, stent-grafts, grafts, filters, occlusive devices, and valves may be made of poly(alpha-hydroxy acid) such as poly-L-lactide (PLLA), poly-D-lactide (PDLA), polyglycolide (PGA), polydioxanone, polycaprolactone, polygluconate, polylactic acid-polyethylene oxide copolymers, modified cellulose, collagen, poly(hydroxybutyrate), polyanhydride, polyphosphoester, poly(aminoacides), or related coploymers materials, each of which have a characteristic degradation rate in the body. For example, PGA and polydioxanone are relatively fast-bioabsorbing materials (weeks to months) and PLA and polycaprolactone are a relatively slow-bioabsorbing material (months to years).

Stents as described are used in the treatment of various medical conditions. One such condition, carcinomas in the esophagus may lead to progressive dysphagia, i.e. difficulty in swallowing, and the inability to swallow liquids in the most severe cases. While surgical removal of the carcinoma is sometimes effective, the majority of patients have tumors that can not be surgically removed. Repeated dilations of the esophagus provide only temporary relief.

Difficult or refractory cases of carcinomas often are treated by intubation using rigid plastic prostheses, or laser therapy with an Nd:YAG laser. These techniques, while often effective, have disadvantages. Rigid plastic prostheses are large, for example having a diameter of 10-12 mm and larger (25-29 mm) outer end flanges. Placement of rigid plastic stents is traumatic, and too frequently causes perforation of the esophageal wall. These prostheses further are subject to migration, obstruction with food or tumor ingrowth, and damage to surrounding cells.

Laser therapy is expensive, typically requiring several treatment sessions. Tumor recurrence is frequent, in the range of 30-40 percent. Submucosal tumors, and certain pulmonary and breast tumors causing dysphagia by esophageal compression, can not be treated by laser therapy.

Patients with benign tumors may also be treated with repeated dilatations using a balloon catheter or a bougie tube. Another treatment approach is submucosal resection. However, violation of the lumen wall carries the risk of wound contamination, as well as possible fistula formation. Following any treatment that alters the lumen wall, the lumen wall remains very sensitive during the healing process. The healing lumen wall can be repeatedly irritated by stomach contents refluxing into the esophagus or a passing food bolus. In addition, surgery is determined based on the absence of certain factors which significantly increase the risk of surgical mortality, morbidity, and long term adverse events. Factors such as cardiac risk, multisystem failure, general debility, malnutrition and infection limit the patient's health and chances of tolerating the radical curative surgical procedure. Thus, esophageal resection with reanastomosis is most appropriate only for very large tumors, annular tumors, or those densely adherent to larger areas of the lumen wall. Tumors at the anastomotic site often reocclude the esophagus and require the same treatments. Pulmonary resections have similar complications.

The search for a more suitable prosthesis has lead to experiments with Gianturco stents, also known as Z-stents. U.S. Patent No. 4,800,882 (Gianturco) describes such a device employed as an endovascular stent. Such stents for the esophagus have been constructed of 0.018 inch stainless steel wire, and provided with a silicone cover to inhibit tumor ingrowth. It was found necessary, however, to provide a distal silicone bumper to prevent trauma to the esophageal lumen wall.

Self-expanding mesh stents also have been considered for use as esophageal prostheses. U.S. Patent No. 4,655,771 (Wallsten) discloses a mesh stent as a flexible tubular braided structure formed of helically wound thread elements. Mesh stents are unlikely to lead to pressure necrosis of the esophageal wall. With its inherent pliability the mesh stent, as compared to a rigid plastic stent, is insertable with much less trauma to the patient. Further, the stent can mold itself to, and firmly fix itself against, the esophageal wall to resist migration.

Thus, both malignant and benign strictures of the esophagus and pulmonary tree may be treated using self-expanding metal stents (SEMS). SEMS allow patients to return to a more normal diet thereby enhancing their quality of life. Generally, benign strictures are treated with SEMS only as a last resort. However, a major complication in both malignant and benign case is stent/lumen re-occlusion over time. That is, the stent is subject to tumor ingrowth because of the spaces between adjacent filaments. This is due, at least in part, to the need to combine sufficient radial force with some open stent mesh to allow tissue incorporation so as to anchor the stent in place. As tissue grows through the mesh (in-growth), and around the stent ends (overgrowth), the body lumen often becomes re-occluded over time.

Stents may also be covered with various materials to encourage or inhibit tissue attachment to the stent. Covered stents are gaining favor for biliary applications because they more effectively inhibit tissue attachment, intrusion, and constriction of the tract than bare stents. For example, polytetrafluoroethylene (PTFE) covered stents are desirable for removable stents because tissue attachment or in-growth is reduced in comparison to bare stent or a stent covered with textile (polyester) material. Laminated ePTFE may also be used to cover stents. U.S. Patent No. 5,843,089 of Sahatjiian et al. describes a stent coated on its inner surfaces with hydrogel (i) to protect cells of the lumen which may have been damaged during deployment of the stent, (ii) to reduce flow disturbances, and (iii) for the delivery of therapeutic agents embodied in the gel.

As stents are covered with material to aid in their removal, stent, migration from the treatment site increases. There remains a continuing need for covered stents which include characteristics to maintain the stent in position at the treatment site. For example, stents covered with ePTFE, such as Precedent, are easily removed after a given time period, such as six months, but may not provide sufficient fixation to prevent the risk of migration during the six month period. U.S. Patent Application Publication No. US2002/0177904 describes a removable stent having a bioabsorbable or biodegradable polymeric outer coating that maintains a helical configuration of the stunt for some period of time. ,Upon degradation or absorption of the coating, the stent is converted back into a soft, elongated shape. U.S. Patent Application Publication No. US2002/0002399 describes another removabable stent structure including an outer bioabsorbable/degradable coating providing rigidity for some period of time afterwhich the stent reverts to a softened filament for removal U.S. Patent No. 5,961,547 describes a similar temporary stent structure.

US 2002/165601 A1 describes a bioabsorbable stent-graft and covered stent comprising an outer element and an inner element. Furthermore it is described that a middle layer comprises a mechanism for attaching the graft portion to the stent portion comprising a continuous adhesive over the outer surface of the stent and the inner surface of the graft that binds them together.

US 5,961,547 is related to a temporary stent for temporary placement within a body. The described stent utilises a removable coil or the like of reinforcing filament such as metal or plastic wire enclosed at least partially within a shell or within a covering of biodegradable/bioabsorbable material which is allowed to remain within the body after removal of the reinforcing filament. More particular a layer is in contact with a vessel with an inner layer made of a bio material that may be softened or liquefied at an elevated temperature. A reinforcing coil is carried within the tubular configuration of the stent and may be removed by pulling it out.

US 6,214,040 relates to sandwich stent with spiraling bands on an outer surface, In particular a stent assembly is described with an inner continuous stent, a fabric cover folded over the inner stent in a collapsed state, an outer stent having a lesser longitudinal length than the inner stent.

US 5,797,949 relates to an apparatus for repairing a body passageway and includes a catheter having first and second inflatable portions, a prosthesis having first and second ends, and a first securing member associated with the first end of the prosthesis and disposed in the second inflatable portion of the catheter.

US 6,254,632 describes an implantable medical device having protruding surface structures for drug delivery and cover attachment. More detailed the stent comprising: a body having a generally cylindrical shape and an outer surface, wherein said outer surface includes a plurality of protruding structures, wherein said protruding structures, include: a depression region having a bottom surface, said depression region approximately centrally located in said protruding structure; and a lip surrounding at least a portion of said depression region, said lip having a height that extends above said bottom surface and said outer surface.

The present invention is disclosed in independent claims 1 and 20 annexed hereto. The dependent claims describe preferred embodiments of the invention.

### SUMMARY OF THE INVENTION

The present invention is directed to a composite stent having more than one distinct and separable elements or members - for example an outer stent element (or outer element) and an inner stent element (or inner element). The properties of the two stent elements may be designed or adjusted to provide the composite stent with desirable properties. For example, and without limitation, one embodiment of the present invention is directed to a composite stent having an outer stent element that remains for a longer period of time in a body lumen and a temporary inner stent element removeably attached to and covering an exposed inner wall surface of the outer element. The outer element, may be, for example, a bioabsorbable stent typically constructed of a relatively non-resilient material such that the outer bioabsorbable stent may not be self-expanding and subject to migration within the lumen over time. In contest, the inner elements may be, for example, and without limitation, a removable self-expanding metal stent (SEMS) used to urge and maintain the position of the outer element in the body lumen. The temporary inner SEMS may retain the composite structure (including the underlying inner element) in position until such time as the outer element is appropriately incorporated into the surrounding tissue or some other criteria occurs such that the removal of the SEMS is indicated. The SEMS may then be detached from the outer element and removed from the body lumen.

Additionally, while the outer stent element and inner stent element may be positioned within the body lumen simultaneously, the present invention is broad enough to cover the positioning of the outer stent element in the body lumen first and the subsequent positioning of the inner stent element *in vivo* to form the composite stent *in vivo.*

Each of the stent elements of the composite stent may also include one or more coverings. A covering may be included to aid in retaining the element in position, maintaining the proper position between stent elements, identifying the locution of the composite stent, preventing tissue in-growth into the stent elements, or introducing medicines or fluids within the patient, for example, as the covering is degraded.

Although the inner element may be a SEMS, other temporary structures may be used to urge the outer element into position for some period of time while providing for normal functioning of the body lumen during such period (e.g., passage of a bodily fluid through both elements). Thus, for example, the inner element may itself be urged into position by a balloon (or other mechanical dilator), thereby anchoring the outer element in position. After a suitable period of time, the inner element may be detached from the outer and removed. Alternatively, the inner element may be made of a biodegradable material such that it is dissolved and/or absorbed by the body over some period of time after which the outer element has been incorporated into the lumen walls.

Thus, according to one aspect of the invention, a composite stent includes an outer element open at opposite ends and having an outer surface engageable with an inner surface of a body lumen. An inner element is likewise open at opposite ends with the inner element engageable with the outer element to form a composite structure (composite stent) insertable within the body lumen. The inner element is configured to maintain the position the outer element within the body lumen.

According to another feature of the invention, the outer element comprises a bioabsorbable stent material, while the inner element may comprise a self-expanding metal stent (SEMS) covered by the outer element. The inner SEMS may be removeably positionable within the outer element so as to provide for removal of the SEMS from the body lumen independent of the outer element. The outer element may comprise (i) a mesh; (ii) a graft; (iii) a tube; (iv) a stent or (v) a similar structure. The inner and outer elements may be attached to each other by a non-biodegradable element such as (i) sutures, (ii) clips, (iii) staples, (iv) an adhesive, and (v) a mechanical interlock. Alternatively, attachment may be accomplished by a bioabsorbable element.

According to another aspect of the invention, a stent includes a bioabsorbable stent element; and a self-expanding metal stent (SEMS) element releasably engageable within the bioabsorbable stent element to form a composite structure for insertion within the body lumen separately or as a unit. The bioabsorbable stent element may be biased to position the outer element into engagement with the body lumen. The bioabsorbable stent element may be made of a bioabsorbable polymer.

According to another aspect of the invention, a method of treatment comprises the steps of inserting a composite stent structure into a body lumen, the composite stent structure including an inner element attached to an outer element; expanding the inner element to cause the outer element to be positioned into contact with an inner wall of the body lumen; and allowing for normal functioning of the body lumen by transporting a bodily substance through the composite stent structure.

These and other objects, advantages and novel features of the invention will be set forth in part in the description which follows, and in part will become apparent to those skilled in the art upon examination of the following or may be learned by practice of the invention. The objects and advantages of the invention may be realized and attained by means of the instrumentalities and combinations particularly pointed out in the appended claims.

### BRIEF DESCRIPTION OF THE DRAWINGS

We first briefly described the drawings.

FIGURE 1 is a diagram of a stent delivery system including a partially deployed stent;

FIGURE 2 is a diagram of a Percutaneous Transluminal Angioplasty (PTA) or Transluminal Coronary Angioplasty (PTCA) balloon being used to expand a stent within a body lumen;

FIGURE 3 is a diagram of an embodiment of the present invention including an inner self-expanding metal stent (SEMS) element located within an outer knitted bioabsorbable stent element, both of which are in a compressed state;

FIGURE 4 is a diagram of the inner SEMS element and outer knitted bioabsorbable stent element embodiment of FIGURE 3 in an expanded state such as in position in a body lumen;

FIGURE 5 is a cross sectional view of an embodiment of the present invention including an outer bioabsorbable element positioned within a removable inner element in situ including retrieval loops for removal of the inner element;

FIGURE 6 is a partial cross sectional view of an embodiment of the present invention which includes an inner and outer stent elements in situ, the outer element including means for accepting in situ application and/or replenishment of a therapeutic agent;

FIGURE 7 is a diagram of an embodiment of the present invention of a composite stent which includes an integral reservoir of a therapeutic agent fluid and a bioabsorbable needle delivery system;

FIGURE 8 is a diagram depicting alternate outer stent element configurations;

FIGURE 9 is a sectional view of an embodiment of the present invention of a composite stent which includes incorporating a fluid reservoir held in place in a body lumen by axial bands of tissue adhesive;

FIGURE 10 is a diagram of an embodiment of the present invention which includes a bioabsorbable outer stent attached to an inner element by sutures;

FIGURE 11 is a diagram of an embodiment of the present invention which includes a bioabsorbable outer stent attached to an inner element by tabs or clips; and

FIGURE 12 is a cross sectional view of an embodiment of the present invention which includes mating surfaces of inner and outer elements having a threaded configuration for retaining one inside the other.

### DETAILED DESCRIPTION

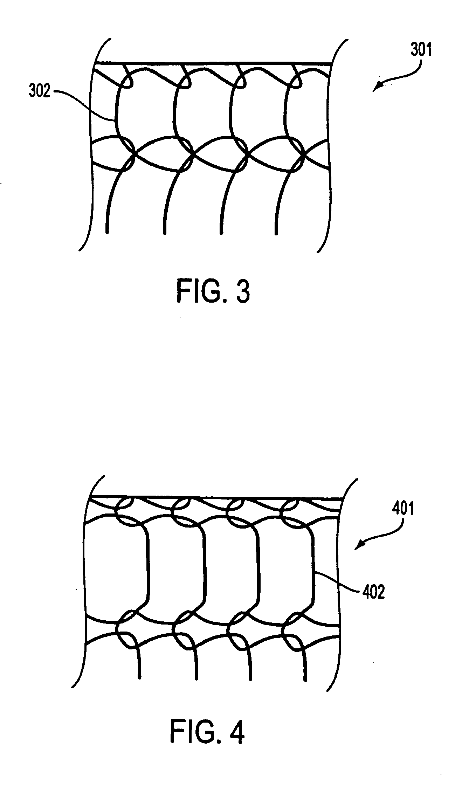

Referring to Figure 3, according to an embodiment of the invention, a composite stent 301 includes an outer bioabsorbable mesh or similar stent element 302 affixed to a fully covered inner self-expanding metal stent (SEMS). Suitable outer bioabsorbable or biodegradable stents are typically made from a bioabsorbable polymer. Polymer structures typically have a higher potential to creep (i.e., experience permanent deformation and fail to return to an original shape and/or size when released) if held in a constrained condition while in the delivery system. The potential for creep in the outer element may increase with temperature elevation such as in sterilization. The fully covered SEMS will self-expand to SEMS as shown in Figure 4 so that the combined structure 401 (including bioabsorbable mesh 402) overcomes any loss in recovered diameter. While some bioabsorbable shape memory polymers may minimize creep, the instant composite stent design simplifies the bioabsorbable material demands. Another advantage of the present invention is that the outer element is not required to support the lumen walls by itself. The inner element may assist the outer element in this respect. Therefore, the outer element may have a lower profile, such as a smaller diameter filament or a flat filament. Through the interaction of the inner element and the outer element the final body lumen diameter, with the stent in place, will have a larger diameter.

This general composite structure provides several advantages. For example, a radiopaque (RO) substance is often added to a stent to assist in identifying the position of the stent within the body lumen. Without the inner covered SEMS, the bioabsorbable component of the stent would need to be loaded with a RO substance to enable fluoroscopic visualization upon deployment. Unfortunately, addition of RO substances to the polymer weakens the polymer thereby limiting the radial strength of the device, and leaving behind a potentially undesirable residual substance when the bioabsorbable element degrades. However, in various embodiments of the present invention, a composite stent may be configured to place the radiopacity into the inner element or a covering of the inner element. This may be done by making an element of the stent of a RO material, placing markers within the element or the covers, incorporating a RO core within an element or by similar methods.

Once the composite stent structure is in place, the bioabsorbable outer stent will, over time, become incorporated into the lumen wall which will keep the combined structure from migrating. The outer element of the present invention may also provide interference or friction to prevent migration prior to integration into the lumen wall. Other methods of preventing migration included within the present invention include hooks or anchors on either stent or cover, adhesives to attach to the vessel wall, designing the outer stents with bumps or ridges or a unique cross-section, suturing or fastening the stent in place in the body, flaring the ends; having retainer rings of larger diameter included at the end of the stents and similar methods and devices.

Addressing the inner element, while any stent element may be used for the inner element, Nitinol SEMS are known to have sufficient radial force and to apply a gradual pressure against the force of the stricture and lumen wall. The bioabsorbable/removable SEMS structure retains the gradual pressure advantage of SEMS that may be compromised with a bioabsorbable stent alone. To obtain a radial force like that of SEMS, a much thicker filament would otherwise be required. The present composite stent technology minimizes the formation scar tissue and allows for the use of more flexible bioabsorbable structures with smaller diameter bioabsorbable filaments. An inner stent cover may be included to provide a barrier to incorporation of the inner stent which enables its eventual removal. According to one embodiment, a fully covered inner section may be removed immediately (within the first day), acutely (within 1-21 days), or chronically (greater than 21 days) following placement of the outer member. The bioabsorbable element or the inner element may be used to fully deploy the outer element, thus avoiding the use of a balloon or other mechanical dilator. In addition to assisting in positioning the outer element, a fully covered SEMS shields the healing lumen wall from recurrent injury associated with stomach acid reflux, food, fluids or other substances that travel through the lumen. This in turn may reduce the amount of scar tissue formed on the lumen wall. Further, tissue buildup is limited to the bioabsorbable filament thickness which defines the gap between the lumen wall and cover.

The combined structure of the composite stent enables removal of the inner element to leave behind only the temporary - absorbable element. The two may be attached by a nondegrading ("permanent") or bioabsorbable means such as sutures, clips, staples, dissolvable gel, adhesive or mechanical interlock. Connectors incorporating easily removable means may also be used such as interwoven filaments which may be pulled out, a crochet that may be unraveled or an inner element which may be "unscrewed" from an outer element. The connection may be made at the extremes of the stents (i.e. through the last row of loops or cells) or any where along the length of the structure. The two may be separated by mechanical means such as a snare, scissors, forceps, laser or a combination of these to sever the connecting component. Alternately, they can be separated through absorption if a bioabsorbable connector is used such as a dissolvable adhesive or a pH reactive connector.

When certain material is chosen, the bioabsorbable backbone, typically the outer element, will become fully incorporated into the lumen wall within approximately four weeks. Typically, once the bioabsorbable stent elements are incorporated, scar tissue will be formed that surrounds and eventually replaces the stent to support the lumen. To accomplish this the bioabsorbable-polymer stent must be in intimate contact with the lumen wall to allow for incorporation. If the stent does not fully expand against the lumen wall or cannot resist the external load from the stricture during healing the lumen will become occluded and dysphagia will return. The inner element such as the SEMS pushes and keeps the bioabsorbable backbone in contact with the lumen wall to promote healing without requiring the bioabsorbable structure to take the full load or gradually expand the lumen. Alternately, the inner element may be balloon expandable. After the incorporation time period, once the site has fully healed, the fully covered inner SEMS may be removed.

The outer bioabsorbable element may be in a form other than a stent mesh. A graft, tube, stent or similar structure may be attached to the inner element to enhance the function of the combined structure. Likewise, the inner element may be in a form other than a stent mesh. In one embodiment, any expandable structure may be used to self-expand the combined structure. Examples may be but are not limited to a dialator, vena cava filters, venous valves Gastroesophageal valves, etc.

The materials used for the inner and outer elements may be reversed. That is, the inner element may be made bioabsorbable or degradable and the outer element a non-absorbable material. This may be desirable where the permanent implant lacks the necessary integrity by itself to resist loading prior to incorporation and/or where a secondary procedure to remove the implant is not possible or desirable. Such a bioabsorbable inner backbone may include elements that are non-absorbable designed to continue to function after removal of-the inner element and/or the bioabsorbable element has degraded. Examples of this may be mechanisms such as valves for antireflux control of stomach contents back into the esophagus, mechanisms such as valves to control reflux of blood from the arterial to venous vessels in the circulatory system (i.e., arterial-venous fistulas in the arm or legs), mechanisms such as valves for the venous system to address DVT. Similarly, use of the outer covering on the inner element will facilitate the same protection of the healing tissue with an alternate outer structure.

In some embodiments the composite stent structure may also be used as a means for agent delivery. The outer bioabsorbable element, the inner element cover or the filament material used on either may be impregnated or coated with an agent in a coating or gel form. This may include outer or inner elements with agents and means of deploying those agents. Such means include, but are not limited to: agent directly on the device, agent within coating of the device (coating being either eluting or responding to triggers such as pressure, sponge, or body heat), device with channels, reservoirs, pores or means to hold agents, the agent within degradable structures such as the device itself of the coating on the device, agents applied by other devices such as delivery catheter or balloon, devices with reservoirs wrapped around, agents within the attachment means, agents released by deployment of either device (cracks open sheath). Further, various coatings may be used to improve the radiopacity, alter the lubricity, surface texture or as means to form the cover in the internal SEMS element. All of these offer a means to improve the function, imaging, therapeutic value, and/or manufacturability of the device. A preferred embodiment for agent deliver is a coated outer stent.

According to another embodiment of the invention, the form of the outer element may be modified to assist in the application of agents. These alternate forms of the outer element may be made to contact with or penetrate the lumen wall. Accordingly the outer element may be made blunt or sharpened depending upon the desired intent. Additionally, the form of the outer element may assist in stabilizing the composite stent in place, or increase its therapeutic value by delivering a great quantity of agent.

Attachment of the inner and outer element may be accomplished using various means, structures and techniques. For example, the inner and outer elements may be attached during manufacturing or deployed separately and attached in-vivo. Various attachment means may also be used. For example, as will be further described, the two may be mechanically interlocked such as by screwing together or alignment of a boss and slot.

The present invention provides several benefits. For example, plastic stents, whether bioabsorbable or of another non-bioabsorbable polymer, usually do not have the radial force of the self expanding metal stents (SEMS) such as Ultraflex^{™} or Wallstent^{R}. The present invention may be used to assist in fully expanding these stents to their intended final diameters once positioned at the site of the stricture.

Further, plastic stents, whether made of a bioabsorbable or non-bioabsorbable material are subject to creep under a sustained load. These stents are often loaded or compressed while preloaded on the delivery system (with or without elevated temperature and humidity associated with sterilization and/or handling). If the stent is held in a constrained configuration where the initial stent diameter is reduced significantly to allow placement into the body, the plastic will likely permanently deform or creep under the load. If a stent has taken a permanent set or other deformity due to packaging and delivery, the size and shape of the stent upon placement into the body of the patient may be incorrect and unsuitable for proper treatment. The present invention may be used to eliminate or reduce creep.

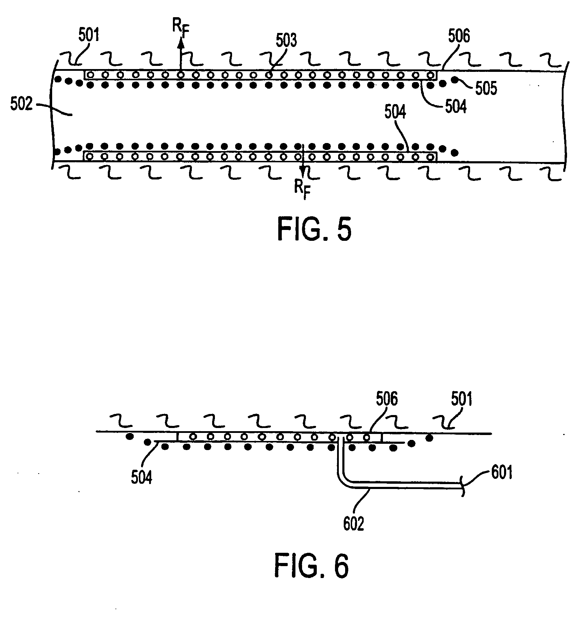

To address the condition where the material creeps due to the load applied during prolonged constrainment on the delivery system and/or due to the sustained and potentially increasing (in time) load from the tumor or stricture the bioabsorbable stent can be affixed to a removable stent. According to one embodiment of the invention as shown in Figure 5, composite stent 502 includes a polymeric outer element 503 which is detachably mounted onto a SEMS inner element 505 forming an inner covering over outer element 503. The inner SEMS element applies a sustained outward radial force F_{R} on a stricture in the lumen or tumor present in the surrounding lumen wall 501 to maintain or eventually achieve the desired body lumen diameter. The SEMS is selected to have a radial force F_{R} sufficient to push the stricture outwardly to open the lumen or vessel.

SEMS used as inner element 505 may be left in place for a period of time to allow the polymeric outer stent element 503 to become incorporated into body lumen wall 501. The typical time range for incorporation of a stent into a vessel or lumen wall is one to three weeks, but may vary depending upon a number of parameters, including materials, geometry, tissue type and condition and force on the tissue

SEMS inner element 505 may include covering 504 over the length upon which the polymeric stent outer element 503 is held. The covering formed over inner element 505 functions to block the tissue from incorporating into the removable SEMS and confine the ingrowth to incorporate the bioabsorbable stent outer element 503. With tissue incorporation around the polymeric stent (outer element 503) and not into the SEMS (inner element 505), the SEMS may be more easily removed with less tissue damage.

The SEMS serves multiple purposes. Upon deployment, the SEMS carries the outer stent element with it through its self expansion and helps to deploy the outer stent element. This avoids the need for using a balloon catheter to deploy the outer stent element as shown in and described in Figure 2. Further, the SEMS maintains a constant radial force against the stricture or lesion. Should the outer stent element not be able to exert a constant positive force against the stricture the SEMS could compensate for this by providing additional outward radial force against the walls of the body lumen.

The SEMS may be removed after the outer stent element has been incorporated into the wall. Once incorporation has occurred, the vessel will be less likely to reduce in size as scar tissue creates a scaffold to hold the lumen or vessel to the desired size.

The outer stent element may be held to the SEMS using a dissolvable gel that adheres the outer stent element to the covered SEMS, or by bioabsorbable or biodegradable sutures, clips or staples or by an adhesive that has a low break away strength. Additionally, biodegradable adhesives, bosses, triggerable dissolution connections may be used to connect the inner and outer elements. Electrical, thermal, light energies, chemical activation and other triggering methods may be used.

In another embodiment of the present invention, either the inner stent element or the outer stent element may be include radiopaque characteristics. One manner of providing radiopacity to either of the stent elements is by use of radiopaque fillers. Radiopaque fillers include compounds such barium that may be mixed integrally or coated on the stent materials. In some situations, fillers may not function optimally; they may compromise the physical characteristics and performance of a device or may be undesirably released into the body. Preferably, the radiopacity of the device is provided by virtue of the innate material properties. In one such embodiment, the SEMS inner stent element may provide sufficient radiopacity to the otherwise radiolucent polymeric outer stent element. In further embodiments, radiopacity may be imparted to the composite stent device by addition of radiopaque filaments or structures within the radiolucent outer stent element. In some embodiments, one or more radiopaque markers are added to either of the stent elements. An alternative to fillers would be a tracer filament or stent within the bioabsorbable or polymeric stent. This is done by using a metallic wire or marker attached or incorporated into the stricture. This of course results in this material being incorporated into the lumen wall or endothelium.

A further advantage of the retrievable SEMS with a bioabsorbable element system is to enable the ability to deliver and localize therapeutic agents (agents) or other, e.g., radioactive seeds.

The bioabsorbable stent and/or SEMS cover may be impregnated, compounded or coated with an agent to enable a very localized delivery of agents to the lumen wall or vascular wall. The SEMS applies a radial force to keep the bioabsorbable stent element in contact with the surrounding lumen wall to allow agent or therapeutic agent uptake. The force may also be used to push the therapeutic agent into the surrounding lumen wall. Additionally, if configured as a retrievable stent, the SEMS may be removed when the therapeutic agent has been delivered or replaced with another stent element comprising a therapeutic agent to affect another cycle of administration. Further, the covered SEMS, if covered with a outer stent element that has been doped, impregnated compounded or coated with a therapeutic agent, would shield the outer element from bodily fluids that might otherwise displace the therapeutic agent. Thus, as shown in Figure 6, using a bioabsorbable or polymeric structure on the back of the SEMS provides an integrated agent delivery-reservoir system. As shown therein, a cover 504 may include dissolvable gel 506 into which a therapeutic agent 601 may be injected through line 602. Therapeutic agent 601 is then forced into the surrounding lumen wall or endothelium 501 by the radial force expressed by inner removable stent 505. Thus, according to this configuration, a reservoir is formed into which therapeutic agents may be loaded. The agents may be delivered to recharge the reservoir via an injection by needle or catheter or by use of an agent delivery balloon attached to a catheter. In a further embodiment, it is possible to replace the inner stent element with another inner stent element comprising a therapeutic agent as illustrated in Figure 5.

Covering 504 on the SEMS of Figure 6 may be used to create a barrier to hold a therapeutic agent and isolate the body lumen from passing bodily fluids (e.g. stomach acid) or gases. Covering 504 may extend the length of the element or a portion thereof. The outer stent element if formed with a mesh consistency (woven, braided, knitted or other) may hold the therapeutic agent with the wall of the outer stent elements or between the inner stent element and the outer stent element. According to an alternative embodiment, as the bioabsorbable element on the body of the SEMS dissolves, the resulting space remaining may be replaced or filled with the therapeutic agent. This allows the body lumen wall to be treated further with the therapeutic agent even in situations where scar tissue may have formed around the outer stent element.

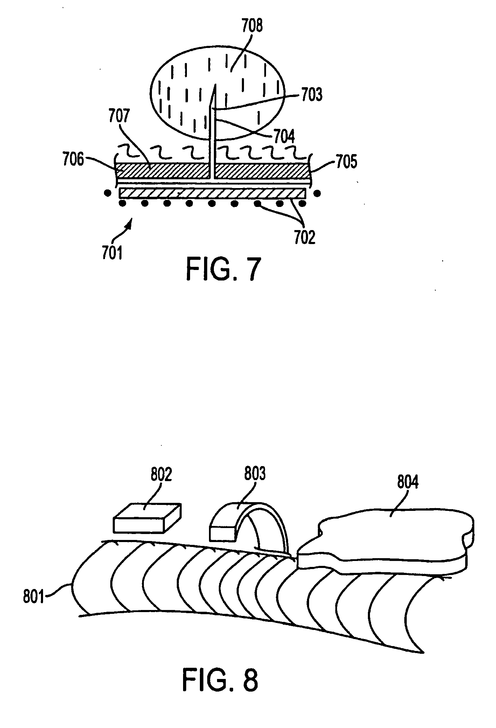

In alternate embodiments, as shown in Figure 7, inner stent element 701 includes a bioabsorbable element 702 to enhance the administration of agents to the body lumen wall. In some embodiments, bioabsorbable element 703 is a needle. In alternate embodiments, element 703 is a protrusion into the body lumen wall or fibers capable of drawing agent 707, stored in reservoir 706 towards the lumen wall. For example, as shown in Figure 7, bioabsorbable needle 703 may be configured to "wick up" 704 through needle 703 a therapeutic agent 707 in the form of a fluid stored in reservoir 706 contained within container 705 and inject the agent into tumor 708.

The inner stent element may also be equipped with a bioabsorbable filament which gives a physician access, through the lumen wall, into tissue below the surface. This access may give the physician a conduit to the underlying tissue (or tumor) as the polymer breaks down. In one embodiment, as the material breaks down the material may be replaced with the therapeutic agent. In this embodiment, the positive force from the inner stent element would push the therapeutic agent to the intended site. A reservoir to hold the therapeutic agent may be formed of a bioabsorbable or pressure sensitive weeping type membrane sack to allow the therapeutic agent to ooze out of the reservoir. In this and other configurations, a needle could serve to wick a therapeutic agent. Alternatively, the body of a needle may comprise a therapeutic agent which is delivered as the needle degrades.

Figure 8 illustrates alternative biodegradable structures that may be positioned at a treatment site and held in place by SEMS 801 until incorporated into the surrounding tissue. In addition to a sack-like reservoir or a weeping reservoir 804, therapeutic agents may be delivered to the body lumen wall by use of agent delivery devices located external to the outer stent element. Such devices include, but are not limited to, a film or other wrapping, one or more bands 803 extending substantially around the circumference of the outer stent element or one or more clips 802 which may deliver a localized amount of agent depending upon its position on the outer stent element.

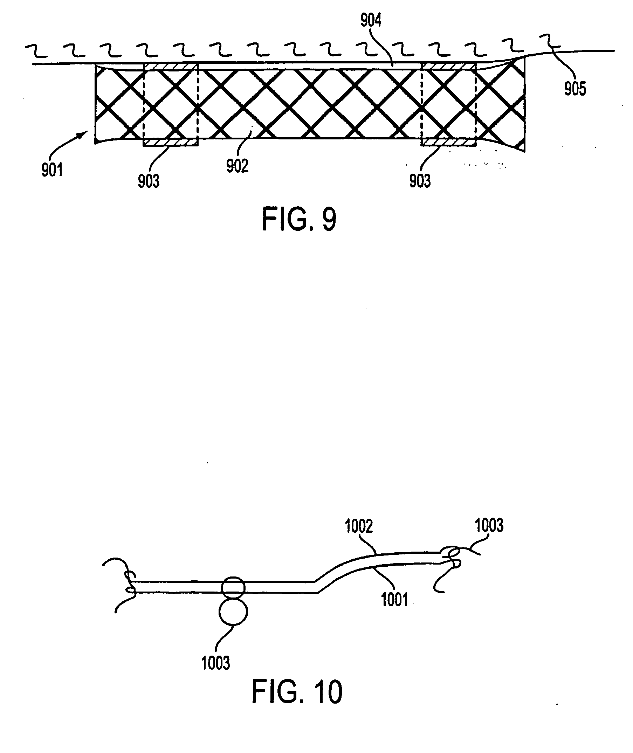

Figure 9 illustrates another embodiment 901 in which a reservoir for holding a therapeutic agent is formed by a cavity created between the stent and the body lumen wall using a covered SEMS. Cover 904 of element 902 may form a reservoir impregnated with or covered with an agent. The contact with body lumen wall 905 could enable transfer while the cover itself would shield the environment. Additionally, the reservoir 904 may comprise a hollow membrane filled with an agent and possibly an agent carrier, a sponge-like material, a hydrogel polymer or similar items. Tissue adhesive 903 may also be included on both ends of the element. _

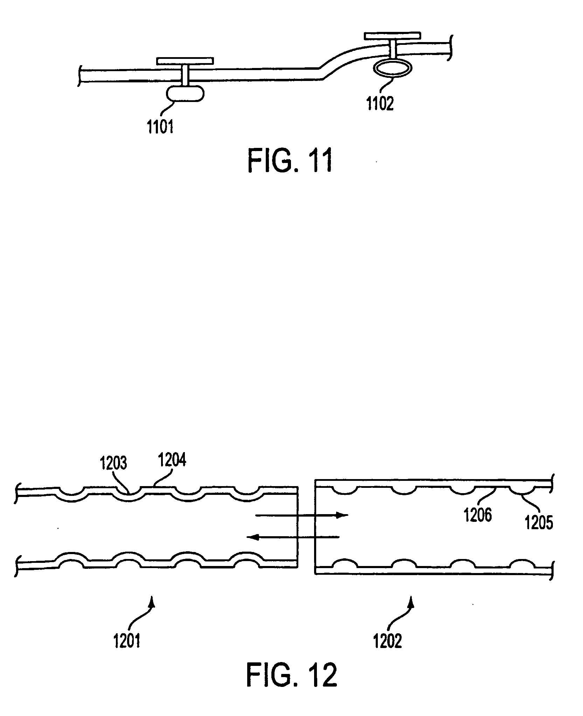

Figure 10 depicts another embodiment in which the bioabsorbable outer stent element 1002 is connected to inner element 1001 using a bioabsorbable or non-bioabsorbable suture 1003 at the extreme ends of the stent or at any point within the length of the two elements. Alternatively, a third intermediate layer may be positioned between the outer stent element and the inner stent element to cause the stent elements to remain intact. This intermediate layer may include grooves, lands or other features to maintain contact between the stent elements. Additionally, the inner and outer element may be interwoven at specific points, preferably with a degradable filament which would allow the elements to be separated at a later time. In one embodiment, one or more sutures may be used to connect the outer stent element to the inner stent element. Using scissors or cutting tool, the suture may be severed and pulled out. Alternatively, sutures 1003 illustrated in Figure 10 may be replaced with tabs 1101 or clips 1102 to connect the two elements as shown in Figure 11. According to other embodiments of the invention, the inner and outer elements may be mechanically interlocked using still other means. For example, the two may be screwed together as shown in Figure 12 wherein locking structures, such as helical grooves or threads, are formed on mating surfaces of the elements. Additionally, inner stent element 1201 may include a configuration of grooves 1203 and lands 1204 configured to mate with respective lands 1205 and grooves 1206 of outer biodegradable element 1202. Likewise, the two elements may be mated together by a dovetail-like connection (not shown). By utilizing the inner covered element to limit tissue incorporation around the element the two elements may be easily unscrewed or disconnected even after an extended period.

Although the present invention has been described with reference to preferred embodiments, those skilled in the art will recognize that changes can be made in form and detail without departing from the spirit and scope of the invention. It will be evident from considerations of the foregoing that the devices of the present invention may be constructed using a number of methods and materials, in a wide variety of sizes and styles for the greater efficiency and convenience of a user.

While the foregoing has described what are considered to be preferred embodiments of the invention, it is understood that various modifications may be made therein and that the invention may be implemented in various forms and embodiments, and that it may be applied in numerous applications, only some of which have been described herein. It is intended by the following claims to claim all such modifications and variations which fall within the true scope of the invention.

## Claims

1. A composite stent comprising:
an outer stent (302; 402; 503; 802, 803, 804; 904; 1002) being open at opposite ends and having an outer surface engageable with an inner surface of a patient body lumen (501; 905); and
an inner stent (505; 701, 702; 801; 902; 1001; 1201) being open at opposite ends, said inner stent (505; 701, 702; 801; 902; 1001; 1201) being engageable with said outer stent (302; 402; 503; 802, 803, 804; 904; 1002) to form a composite structure (301, 302; 401, 402; 502, 503, 505; 701, 702; 801, 802, 803, 804; 901; 902; 904; 1001, 1002; 1201, 1202) insertable within the body lumen (501; 905), said inner stent (505; 701, 702; 801; 902; 1001; 1201) configured to assist said outer stent (302; 402; 503; 802, 803, 804; 904; 1002) in retaining a position of the outer stent (302; 402; 503; 802, 803, 804; 904; 1002) within the body lumen (501; 905);
**characterized in that**
said outer stent (302; 402; 503; 802, 803, 804; 904; 1002) and said inner stent (505; 701, 702; 801; 902; 1001; 1201) are deployed separately and attached in-vivo; and
said inner stent (505; 701, 702; 801; 902; 1001; 1201) is removably attached inside said outer stent (302; 402; 503; 802, 803, 804; 904; 1002) so as to provide for removal of said inner stent (505; 701, 702; 801; 902; 1001; 1201) from the body lumen (501; 905) independent of said outer stent (302; 402; 503; 802, 803, 804; 904; 1002).

2. The composite stent of claim 1, wherein said inner (505; 701, 702; 801; 902; 1001; 1201) and said outer (302; 402; 503; 802, 803, 804; 904; 1002) stent are inserted within the body lumen (501; 905) as a unit.

3. The composite stent according to claim 1, wherein one of said inner (505; 701, 702; 801; 902; 1001; 1201) and outer (302; 402; 503; 802, 803, 804; 904; 1002) stent is made of a relatively biodegradable or bioabsorbable material and the other is made of a relatively non-biodegradable material.

4. The composite stent according to claim 1, wherein said inner stent (505; 701, 702; 801; 902; 1001; 1201) is a self-expanding metal stent.

5. The composite stent according to claim 1, wherein said inner stent (505; 701, 702; 801; 902; 1001; 1201) is configured to provide a radially outward bias so as to position said outer stent (302; 402; 503; 802, 803, 804; 904; 1002) into engagement with the body lumen (501; 905).

6. The composite stent according to claim 1, wherein said outer stent (302; 402; 503; 802, 803, 804; 904; 1002) is configured to provide a radially outward bias so as to engage the body lumen (501; 905).

7. The composite stent according to claim 1, wherein said inner stent (505; 701, 702; 801; 902; 1001; 1201) is configured to accept a balloon therein, inflation of the balloon forcing said inner stent (505; 701, 702; 801; 902; 1001; 1201) to expand so as to position said outer stent (302; 402; 503; 802, 803, 804; 904; 1002) into engagement with the body lumen (501; 905).

8. The composite stent according to claim 1, wherein said outer stent (302; 402; 503; 802, 803, 804; 904; 1002) comprises a bioabsorbable stent material.

9. The composite stent according to claim 1, wherein said inner and outer stents are attached to each other by a non-biodegradable element.

10. The composite stent according to claim 10, wherein said non- biodegradable element is selected from the group consisting of (i) sutures, (ii) clips, (iii) staples, (iv) an adhesive, and (v) a mechanical interlock.

11. The composite stent according to claim 1, wherein said inner and outer stents are attached to each other by a bioabsorbable element.

12. The composite stent according to claim 12, wherein said bioabsorbable element is selected from the group consisting of (i) sutures, (ii) clips, (iii) staples, (iv) an adhesive, and (v) a mechanical interlock.

13. The composite stent according to claim 12, wherein said outer stent (302; 402; 503; 802, 803, 804; 904; 1002) is radiolucent.

14. The composite stent according to claim 1, wherein said inner sent (505; 701, 702; 801; 902; 1001; 1201) is radiopaque.

15. The composite stent according to claim 1, wherein said outer stent (302; 402; 503; 802, 803, 804; 904; 1002) comprises a material for receiving an injection of a therapeutic agent with said outer stent (302; 402; 503; 802, 803, 804; 904; 1002) in situ in the body lumen (501; 905).

16. The composite stent according to claim 1, wherein said outer stent (302; 402; 503; 802, 803, 804; 904; 1002) includes a fluid reservoir and at least one needle configured to transport a fluid from said reservoir through the inner surface of the body lumen (501; 905) to an underlying area to be treated.

17. The composite stent according to claim 1, wherein an inner surface of said outer stent (302; 402; 503; 802, 803, 804; 904; 1002) is configured to mate with an outer surface of said inner stent.

18. The composite stent according to claim 1, wherein an inner surface of said outer stent (302; 402; 503; 802, 803, 804; 904; 1002) includes a plurality of lands and grooves configured to engage respective grooves and lands of an outer surface of said inner stent.

19. The composite stent of claim 1 further including a covering on one of said outer stent (302; 402; 503; 802, 803, 804; 904; 1002) and said inner stent.

20. The composite stent of claim 1 whereby said outer stent (302; 402; 503; 802, 803, 804; 904; 1002) is a bioabsorbable stent element;
said inner stent (505; 701, 702; 801; 902; 1001; 1201) is a self-expanding metal stent element releasably engageable within said bioabsorbable stent element for insertion within the patient body lumen (501; 905) as a unit, and said bioabsorbable stent element is biased to position said outer element into engagement with the body lumen (501; 905) by said inner stent (505; 701, 702; 801; 902; 1001; 1201).

21. The composite stent according to claim 21, wherein said bioabsorbable stent element comprises a bioabsorbable polymer.

## Patentansprüche

1. Komposit-Stent, umfassend:
einen äußeren Stent (302; 402; 503; 802, 803, 804; 904; 1002), der an gegenüberliegenden Enden offen ist, und eine äußere Oberfläche aufweist, die in eine innere Oberfläche eines Körperlumens (501; 905) eines Patienten eingreifen kann; und
einen inneren Stent (505; 701, 702; 801; 902; 1001; 1201), der an gegenüberliegenden Enden offen ist, wobei der innere Stent (505; 701, 702; 801; 902; 1001; 1201) in den äußeren Stent (302; 402; 503; 802, 803, 804; 904; 1002) eingreifen kann, um eine Kompositstruktur (301, 302; 401, 402; 502, 503, 505; 701, 702; 801, 802, 803, 804; 901; 902; 904; 1001, 1002; 1201, 1202) zu bilden, die in das Körperlumen (501; 905) eingefügt werden kann, wobei der innere Stent (505; 701, 702; 801; 902; 1001; 1201) gestaltet ist, um dem äußeren Stent (302; 402; 503; 802, 803, 804; 904; 1002) beim Beibehalten einer Position des äußeren Stents (302; 402; 503; 802, 803, 804; 904; 1002) innerhalb des Körperlumens (501; 905) zu helfen;
**dadurch gekennzeichnet, dass**
der äußere Stent (302; 402; 503; 802, 803, 804; 904; 1002) und der innere Stent (505; 701., 702; 801; 902; 1001; 1201) separat eingesetzt und in vivo befestigt werden; und
der innere Stent (505; 701, 702; 801; 902; 1001; 1201) innerhalb des äußeren Stents (302; 402; 503; 802. 803, 804; 904; 1002) entfernbar befestigt ist, um ein Entfernen des inneren Stents (505; 701, 702; 801; 902; 1001; 1201) aus dem Körperlumen (50); 905) unabhängig von dem äußeren Stent (302; 402; 503; 802., 803, 804; 904; 1002) zu ermöglichen.

2. Komposit-Stent nach Anspruch 1, wobei der innere (505; 701, 702; 801; 902; 1001; 1201) und der äußere (302; 402; 503; 802, 803, 804; 904; 1002) Stent innerhalb des Körperlumens (501; 905) als eine Einheit eingefügt werden.

3. Komposit-Stent nach Anspruch 1, wobei einer der inneren (505; 701, 702; 801; 902; 1001; 1201) und äußeren (302; 402; 503; 802, 803, 804; 904; 1002) Stent aus einem relativ biologisch abbaubaren oder biologisch absorbierbarem Material hergestellt ist, und der andere aus einem relativ biologisch nicht abbaubarem Material hergestellt ist.

4. Komposit-Stent nach Anspruch 1, wobei der innere Stent (505; 701, 702; 801; 902; 1001; 1201) ein selbstexpandierender Metall-Stent ist.

5. Komposit-Stent nach Anspruch 1, wobei der innere Stent (505; 701, 702; 801; 902; 1001; 1201) gestaltet ist, um eine radial auswärtige Spannung bereitzustellen, um den äußeren Stent (302; 402; 503; 802, 803, 804; 904; 1002) in Eingriff mit dem Körperlumen (501; 905) zu positionieren.

6. Komposit-Stent nach Anspruch 1, wobei der äußere Stent (302; 402; 503; 802, 803, 804; 904; 1002) gestaltet ist, um eine radial auswärtige Spannung bereitzustellen, um in das Körperlumen (501; 905) einzugreifen.

7. Komposit-Stent nach Anspruch 1. wobei der innere Stent (505; 701, 702; 801; 902; 1001: 1201) gestaltet ist, um einen Ballon darin aufzunehmen, wobei ein Aufblasen des Ballons den inneren Stent (505; 701. 702; 801; 902; 1001: 1201) zum Expandieren zwingt, um den äußeren Stent (302; 402; 503; 802, 803, 804; 904; 1002) in Eingriff mit dem Körperlumen (501; 905) zu positionieren.

8. Komposit-Stent nach Anspruch 1, wobei der äußere Stent (302; 402; 503; 802, 803, 804; 904; 1002) ein biologisch absorbierbares Stent-Material umfasst.

9. Komposit-Stent nach Anspruch 1, wobei die inneren und äußeren Stents durch ein biologisch nicht abbaubares Element aneinander befestigt sind.

10. Komposit-Stent nach Anspruch 10, wobei das biologisch nicht abbaubare Element aus der Gruppe ausgewählt ist, die aus (I) Suturen, (II) Clips, (III) Stapeln, (IV) einem Klebstoff und (V) einer mechanischen Sperre besteht.

11. Komposit-Stent nach Anspruch 1, wobei die inneren und äußeren Stents durch ein biologisch absorbierbares Element aneinander befestigt sind.

12. Komposit-Stent nach Anspruch 11, wobei das biologisch absorbierbares Element aus der Gruppe ausgewählt ist, die aus (I) Suturen, (II) Clips, (III) Stapeln, (IV) einem Klebstoff und (V) einer mechanischen Sperre besteht.

13. Komposit-Stent nach Anspruch 12, wobei der äußere Stent (302; 402; 503; 802, 803, 804; 904; 1002) strahlendurchlässig ist.

14. Komposit-Stent nach Anspruch 1, wobei der innere Stent (505; 701, 702; 801; 902; 1001; 1201) radioopak ist.

15. Komposit-Stent nach Anspruch 1, wobei der äußere Stent (302; 402; 503; 802, 803, 804; 904; 1002) ein Material zur Aufnahme einer Injektion eines therapeutischen Mittelt mit dem äußeren Stent (302; 402; 503; 802, 803. 804; 904; 1002) in situ in dem Körperlumen (501; 905) umfasst.

16. Komposit-Stent nach Anspruch 1. wobei der äußere Stent (302; 402; 503; 802, 803, 804: 904; 1002) ein Flüssiskeitsreservoir und zumindest eine Nadel einschließt, die zum Transport einer Flüssigkeit aus dem Reservoir durch die innere Oberfläche des Körperlumens (501; 905) zu einem daninterliegenden, zu behandelnden Gebiet gestaltet ist.

17. Komposit-Stent nach Anspruch 1, wobei eine innere Oberfläche des äußeren Stents (302; 402; 503; 802, 803, 804; 904; 1002) zum Zusammenpassen mit einer äußeren Oberfläche des inneren Stents gestaltet ist.

18. Komposit-Stent nach Anspruch 1, wobei eine innere Oberfläche des äußeren Stents (302; 402; 503; 802, 803, 804; 904; 1002) eine Vielzahl von Stegen und Nuten einschließt, die zum Eingreifen in jeweilige Nuten und Stege einer äußeren Oberfläche des inneren Stents gestaltet sind.

19. Komposit-Stent nach Anspruch 1, weiterhin eine Abdeckung auf einem der äußeren Stents (302; 402; 503; 802, 803, 804; 904; 1002) und der inneren Stents beinhaltend.

20. Komposit-Stent nach Anspruch 1, wobei der äußere Stent (302; 402; 503; 802, 803, 804; 904; 1002) ein biologisch absorbierbares Stent-Element ist; der innere Stent (505; 701, 702; 801; 902; 1001; 1201) ein selbstexpandierendes Metall-Stent-Element ist, welches lösbar innerhalb des biologisch absorbierbaren Stent-Elements zum Einfügen in das Körperlumen (501; 905) des Patienten als eine Einheit eingreifen kann, und das biologisch absorbierbare Stent-Element gespannt ist, um das äußere Element in Eingriff mit dem Körperlumen (501; 905) durch den inneren Stent (505; 701, 702: 801; 902; 1001; 1201) zu positionieren.

21. Komposit-Stent nach Anspruch 20, wobei das biologisch absorbierbare Stent-Element ein biologisch absorbierbares Polymer umfasst.

## Revendications

1. Un stent composite comprenant :
un stent extérieur (302 ; 402 ; 503 ; 802, 803, 804 ; 904 ; 1002) qui est ouvert à des extrémités opposées et qui possède une surface extérieure pouvant venir en contact avec une surface intérieure d'une lumière corporelle d'un patient (501 ; 905) ; et
un stent intérieur (505 ; 701, 702 ; 801 ; 902 ; 1001 ; 1201) qui est ouvert à des extrémités opposées, ledit stent intérieur (505 ; 701, 702 ; 801 ; 902 ; 1001 ; 1201) pouvant venir en contact avec ledit stent extérieur (302 ; 402 ; 503 ; 802, 803, 804 ; 904 ; 1002) pour former une structure composite (301, 302 ; 401, 402 ; 502, 503, 505 ; 701, 702 ; 801, 802, 803, 804 ; 901, 902, 904 ; 1001, 1002 ; 1201, 1202) qui peut être inséré à l'intérieur de la lumière corporelle (501 ; 905), ledit stent intérieur (505 ; 701, 702 ; 801 ; 902 ; 1001 ; 1201) étant configuré pour aider ledit stent extérieur (302 ; 402 ; 503 ; 802, 803, 804 ; 904 ; 1002) à conserver une position du stent extérieur (302 ; 402 ; 503 ; 802, 803, 804 ; 904 ; 1002) à l'intérieur de la lumière corporelle (501 ; 905) ;
**caractérisé en ce que**
ledit stent extérieur (302 ; 402 ; 503 ; 802, 803, 804 ; 904 ; 1002) et ledit stent intérieur (505 ; 701, 702 ; 801 ; 902 ; 1001 ; 1201) sont déployés séparément et solidarisés *in vivo ;* et
ledit stent intérieur (505 ; 701, 702 ; 801 ; 902 ; 1001 ; 1201) est solidarisé de manière amovible à l'intérieur dudit stent extérieur (302 ; 402 ; 503 ; 802, 803, 804 ; 904 ; 1002) de manière à permettre le retrait dudit stent intérieur (505 ; 701, 702 ; 801 ; 902 ; 1001 ; 1201) hors de la lumière corporelle (501 ; 905) indépendamment dudit stent extérieur (302 ; 402 ; 503 ; 802, 803, 804 ; 904 ; 1002).

2. Le stent composite de la revendication 1, dans lequel lesdits stents intérieur (505 ; 701, 702 ; 801 ; 902 ; 1001 ; 1201) et extérieur (302 ; 402 ; 503 ; 802, 803, 804 ; 904 ; 1002) sont insérés à l'intérieur de la lumière corporelle (501 ; 905) de façon unitaire.

3. Le stent composite selon la revendication 1, dans lequel l'un d'entre ledit stent intérieur (505 ; 701, 702 ; 801 ; 902 ; 1001 ; 1201) et extérieur (302 ; 402 ; 503 ; 802, 803, 804 ; 904 ; 1002) est réalisé en un matériau relativement biodégradable ou bio-absorbable et l'autre est réalisé en un matériau relativement non biodégradable.

4. Le stent composite selon la revendication 1, dans lequel ledit stent intérieur (505 ; 701, 702 ; 801 ; 902 ; 1001 ; 1201) est un stent en métal auto-expansible.

5. Le stent composite selon la revendication 1, dans lequel ledit stent intérieur (505 ; 701, 702 ; 801 ; 902 ; 1001 ; 1201) est configuré pour produire une sollicitation radiale vers l'extérieur de manière à positionner ledit stent extérieur (302 ; 402 ; 503 ; 802, 803, 804 ; 904 ; 1002) en contact avec la lumière corporelle (501 ; 905).

6. Le stent composite selon la revendication 1, dans lequel ledit stent extérieur (302 ; 402 ; 503 ; 802, 803, 804 ; 904 ; 1002) est configuré pour procurer une sollicitation radiale vers l'extérieur de manière à venir en contact avec la lumière corporelle (501 ; 905).

7. Le stent composite selon la revendication 1, dans lequel ledit stent intérieur (505 ; 701, 702 ; 801 ; 902 ; 1001 ; 1201) est configuré pour recevoir un ballonnet à l'intérieur, le gonflement du ballonnet forçant ledit stent intérieur (505 ; 701, 702 ; 801 ; 902 ; 1001 ; 1201) à se dilater de manière à positionner ledit stent extérieur (302 ; 402 ; 503 ; 802, 803, 804 ; 904 ; 1002) jusqu'en contact avec la lumière corporelle (501 ; 905).

8. Le stent composite selon la revendication 1, dans lequel ledit stent extérieur (302 ; 402 ; 503 ; 802, 803, 804 ; 904 ; 1002) comprend un matériau de stent bioabsorbable.

9. Le stent composite selon la revendication 1, dans lequel lesdits stents intérieur et extérieur sont solidarisés l'un à l'autre par un élément non biodégradable.

10. Le stent composite selon la revendication 9, dans lequel ledit élément non biodégradable est choisi dans le groupe formé par : (i) les sutures, (ii) les clips, (iii) les agrafes, (iv) un adhésif et (v) un connecteur mécanique.

11. Le stent composite selon la revendication 1, dans lequel lesdits stents intérieur et extérieur sont solidarisés l'un à l'autre par un élément bioabsorbable.

12. Le stent composite selon la revendication 11, dans lequel ledit élément bioabsorbable est choisi dans le groupe formé par : (i) les sutures, (ii) les clips, (iii) les agrafes, (iv) un adhésif et (v) un connecteur mécanique.

13. Le stent composite selon la revendication 11, dans lequel ledit stent extérieur (302 ; 402 ; 503 ; 802, 803, 804 ; 904 ; 1002) est radiolumineux.

14. Le stent composite selon la revendication 1, dans lequel ledit stent intérieur (505 ; 701, 702 ; 801 ; 902 ; 1001 ; 1201) est radio-opaque.

15. Le stent composite selon la revendication 1, dans lequel ledit stent extérieur (302 ; 402 ; 503 ; 802, 803, 804 ; 904 ; 1002) comprend un matériau pour recevoir une injection d'un agent thérapeutique avec ledit stent extérieur (302 ; 402 ; 503 ; 802, 803, 804 ; 904 ; 1002) *in situ* dans la lumière corporelle (501 ; 905).

16. Le stent composite selon la revendication 1, dans lequel ledit stent extérieur (302 ; 402 ; 503 ; 802, 803, 804 ; 904 ; 1002) comprend un réservoir de fluide et au moins une aiguille configurée pour transporter un fluide depuis ledit réservoir au travers de la surface intérieure de la lumière corporelle (501 ; 905) vers une région sous-jacente devant être traitée.

17. Le stent composite selon la revendication 1, dans lequel une surface intérieure dudit stent extérieur (302 ; 402 ; 503 ; 802, 803, 804 ; 904 ; 1002) est configurée pour venir épouser une surface extérieure dudit stent intérieur.

18. Le Stent composite selon la revendication 1, dans lequel une surface intérieure dudit Stent extérieur (302 ; 402 ; 503 ; 802, 803, 804 ; 904 ; 1002) comprend une pluralité de collets et de gorges configurés pour venir s'engager avec des gorges et collets respectifs d'une surface extérieure dudit stent intérieur.

19. Le stent composite selon la revendication 1, comprenant en outre un revêtement sur l'un dudit stent extérieur (302 ; 402 ; 503 ; 802, 803, 804 ; 904 ; 1002) et dudit stent intérieur.

20. Le stent composite de la revendication 1, où ledit stent extérieur (302 ; 402 ; 503 ; 802, 803, 804 ; 904 ; 1002) est un élément de stent bioabsorbable ; ledit stent intérieur (505 ; 701, 702 ; 801 ; 902 ; 1001 ; 1201) est un élément de stent en métal auto-expansible qui peut être engagé de façon libérable à l'intérieur dudit élément de stent bioabsorbable pour insertion au sein de la lumière corporelle du patient (501 ; 905) de façon unitaire, et ledit élément de stent bioabsorbable est sollicité pour positionner ledit élément extérieur en contact avec la lumière corporelle (501 ; 905) par ledit stent intérieur (505 ; 701, 702 ; 801 ; 902 ; 1001 ; 1201).

21. Le stent composite selon la revendication 20, dans lequel ledit élément de stent bioabsorbable comprend un polymère bioabsorbable.
